# EUROPEAN PATENT APPLICATION

(11) **EP 0 583 976 A2**
(43) Date of publication of application: **23.02.1994**
(21) Application number: 93306487.5
(22) Date of filing: 17.08.1993
(51) Int. Cl.: A61K 47/48, A61K 33/10, A61K 33/08

(54) **Gastroprotective complexes with cyclodextrin**

(30) Priority: 17.08.1992 US 931227
(71) Applicant: Bristol-Myers Squibb Company, New York, N.Y. 10154 (US)
(72) Inventor: Koslo, Randy P., Cranbury, New Jersey 08512 (US)
(74) Representative: Ritter, Stephen David

(57) **Abstract**

Inclusion compounds of cyclodextrins and buffers and methods of their manufacture are disclosed that provide a gastro-protective benefit.

## Description

### Field of Invention

The present invention concerns the treatment of gastrointestinal disorders. More specifically, it concerns such treatment by orally administering a medicinal agent that has been complexed with a cyclodextrin. Most specifically, the invention concerns the wholly unexpected enhancement of gastroprotection afforded by the complexed medicinal agent.

### Background of Invention

Gastrointestinal disorders range in severity from gastritis related to a single, well-defined event to acute ulcers that heal rapidly when the stressful etiology is removed.

Acute gastritis is the most common GI complaint, and is most often initiated by overindulgence of food or alcohol. The disease mechanism appears to be a break in the normal gastric barrier, which allows hydrochloric acid to injure vessels in the mucosa, resulting in hemorrhage, inflammation and erosion. Treatment is with conventional antacids, e.g., sodium bicarbonate, calcium carbonate, aluminum hydroxide, magnesium oxides, and magnesium-aluminum hydroxide gels.

Ulcers -- lesions in the stomach intestine and esophagus and stoma -- arise from many causes, including the presence of ulcerogenics such as alcohol, caffeine, glucororticosteroids, nonsteroidal antiinflammatory drugs, e.g., aspirin and indomethacin, and tobacco; stress, perhaps causing hyperacidity; and abnormal body chemistry. Treatment is with antacids or, in more severe cases, with an antiulcer medication such as sulcralfate and cetraxate hydrochloride.

Antacids neutralize gastric acid, thereby raising gastric pH. At a pH of about 2.3, approximately 90% of the acid will be neutralized and at a pH of about 3.3, approximately 99% of the acid is neutralized. Raising pH inhibits the action of pepsin. While a possible effect of antacids is to tighten the mucosal barrier and increase esophageal sphincter tone, antacids generally do not coat ulcer linings.

However, the literature does consider whether antacids are cytoprotective. Hollander et al, Are Antacids Cytoprotective, Gut 30:145-7 (1989) suggests that aluminum-containing and bismuth-containing compounds stimulate prostaglandin synthesis by gastric mucosa, which is important in gastric micosal defense. See also, Szelenyi et al, Release of Cytoprotective PGE₂ From Cultured Macrophages Induced by Antacids and Sucralfate, Agents and Actions, 18:375-80 (1986); Domschke et al, Antacids and Gastric Mucosal Protection, Scand. J. Gastroeneterol., 21 (Suppl. 125):145-9 (1986); Hollander et al, Protection Against Alcohol-Induced Gastric Mucosal Injury by Aluminum-Containing Compounds -- Sucralfate, Antacids, and Aluminum Sulfate, Scand. J. Gastroenterol., 21 (Suppl. 125):151-3 (1986); Di Joseph et al, Activated Aluminum Complex Derived From Solubilized Antacids Exhibits Enhanced Cytoprotective Activity in the Rat, Gastroenterology, 96:730-5 (1989); Borella et al, Cytoprotective and Antiulcer Activities of the Antacid Magaldrate in the Rat, Arzneim.-Forsch/Drug Res., 39 (II):786-9 (1989), and Pugh et al, Brit. J. Exp. Pathol., 69:833-8 (1988).

The patent literature also makes mention of the role of antacids in the treatment of gastrointestinal disorders. Thus, U.S. 4,857,324 to Mir et al discloses exogenously acidified antacid compositions having cytoprotective properties. Also of interest are U.S. 3,591,680 to Greene et al; U.S. 3,253,988 to Scott; U.S. 3,164,519 to Puetzer et al; U.S. 3,155,576 to Lish et al and U.S. 3,538,214 to Polli et al as disclosing antacid-containing compositions.

U.S. 4,042,685 to Smith is of interest as disclosing a spray-dried product comprising a dessicated mixture of magnesium hydroxide magma and dextrin.

Cyclodextrins are cyclic degradation products of starch which contain six, seven or eight glucose residues and have the shape of large ring molecules. The most important cyclodextrins are α-cyclodextrin ("α-CD") which consists of six anhydro-glucopyranose units, β-cyclodextrin ("β-CD") which has seven anhydroglucopyranose units, and γ-cyclodextrin ("γ-CD") which has eight anhydroglucopyranose units. Cyclodextrin derivatives are also known, e.g., heptakis-(2,6-di-O-methyl)-β-cyclodextrin ("H-DMe-β-CD") and heptakis-(2,3,6-tri-O-methyl)-β-cyclodextrin ("H-TMe-β-CD").

Cyclodextrins are known for their ability to form addition compounds -- clathrates or inclusion compounds -- by inclusion of molecules in cavities present in large molecules.

Cyclodextrins have been used, as illustrated by the patent and journal literature, to form such complexes with a number of medicinal agents, illustrative of which are: Ikeda et al, Inclusion Complexes of β-Cyclodextrin With Antiinflammatory Drugs Fenamates in Aqueous Solution, Chem. Pharm. Bull., 23:201-8 (1975); Kurozumi et al, Inclusion Compounds of Non-Steroidal Antiinflammatory and Other Slightly Water Soluble Drugs With α- and β-Cyclodextrins in Powdered Form, Chem. Pharm. Bull, 23:3062-8 (1975); Nambu et al, Bioavailability of Powdered Inclusion Compounds of Nonsteroidal Antiinflammatory Drugs With β-Cyclodextrin in Rabbits and Dogs, Chem. Pharm. Bull, 26:2952-6 (1978); Koizumi et al, Enhancement of the Hypnotic Potency of Barbiturates by Inclusion Complexes With β-Cyclodextrin, Chem. Pharm. Bull., 28:319-22 (1980); Otagiri et al, Improvements to Some Pharmaceutical Properties of Flurbiprofen by β- and α-Cyclodextrin Complexations, Acta Pharm. Suecica, 20:1-10 (1983); Ukema et al, Improvement of Dissolution and Suppository Release Characteristics of Flurbiprofen by Inclusion Complexation With Heptakis (2,6-di-O-methyl)-β-cyclodextrin, J. Pharm. Sci., 74:841-5 (1985); Chow et al, Characterization, Dissolution and Bioavalability Increase of Ibuprofen-β-Cyclodextrin Complex System, Intl. J. of Pharm., 28:95-101 (1986), and Zecchi et al, Control of the Release From Solid Dosage Forms; NSAID β-Cyclodextrin Complex, Proc.-Eur. Congr. Biopharm. Pharmacokinet., 3rd, 1:526-31 (1987).

Also of interest are U.S. 4,599,327 to Nogradi et al disclosing CD complexes with dibenzo[bd]pyran derivatives; U.S. 4,565,807 to Uenkama et al disclosing pirprofen-CD complexes; U.S. 4,451,457 to Yoshikumi et al disclosing the use of cyclodextrin to promote the proliferation of intestinal bifidobacteria, and JP 56-46837/1981 disclosing a method of manufacture of ibuprofen enclosure compounds.

U.S. 4,727,064 to Pitha discloses pharmaceutical preparations consisting essentially of a drug with substantially low water solubility and an amorphous water-soluble cyclodextrin. The cyclodextrin is made amorphous by nonselective alkylation with, for example, propylene oxide, glycidol, iodoacetamide, chloroacetate or 2-diethylaminoethylchloride to provide a mixture of many cyclodextrin derivatives. The amorphous character of the cyclodextrin derivatives is attributable to the multiplicity of compounds present in the resulting reaction product.

According to Pitha, the compositions are characterized by improved dissolution of the drug, and hence its absorption into the body. Illustrative of the claims of medicinal agents solubilized by Pitha are analgesics (acetaminophen and indomethacin); diuretics (chlorthalidone and furosemide); vasodilators (nitroglycerin and oxprenolol); cardiotonics (degoxin and oubain); vitamins; progestins; estrogens, etc.

### Summary of the Invention

Applicant has found that a substantial gastroprotective benefit is obtained when an antacid agent is complexed with a cyclodextrin. The benefit is also realized with antacid agents that heretofore have not been reported as having a gastroprotective indication. In the case of antacid agents based on, for example, aluminum, Applicant has found that the level of gastroprotection afforded by its complexation with cylodextrin is greatly enhanced. The discovery of Applicant disclosed herein is not only surprising in view of the remarkable gastroprotection results obtained, but is all the more surprising since there are few, if any, reported instances known to Applicant in which an inorganic agent such as used herein forms a complex with cyclodextrin.

### Detailed Description of the Invention

The gastroprotective complex of the present invention comprises an antacid medicinal agent that has been complexed with a cyclodextrin selected from the group consisting of α-, β- or γ-cyclodextrin and their lower alkyl and lower hydroxyalkyl derivatives.

### The Antacid Medicinal Agent

The antacid agents suitable for complexation with the cylcodextrin host molecule are those which are salts of calcium, aluminum and magnesium. While the etiology of the various types of antacid agents may be different, they all appear to provide a buffering action when administered orally, thereby raising stomach pH. Suitable salts are calcium carbonate, aluminum hydroxide, aluminum carbonate, magnesium oxide or magnesium hydroxide, magnesium trisilicate, and magnesium carbonate. Also suitable are aluminum hydroxide hydrates, for example, algedrate; magnesium-aluminum hydroxides, for example, magaldrate, and aluminum-magnesium-silicas, for example, silodrate. The antacid agent is present in the complex in an amount of from about 0.225 to about 60, preferably from about 2.25 to about 30, most preferably from about 10 to about 22.5 mEq of the antacid.

### The Cyclodextrin Host

α-, β- and γ-cyclodextrins are suitable as the host molecule in the complexes of the present invention. Also suitable are the lower alkyl and lower hydroxyalkyl derivatives of these cyclodextrins, especially of the β-cyclodextrin. By "lower alkyl" or "lower hydroxyalkyl" is meant a substituent group having from 1 to about 4 carbon atoms, and preferably is methyl. These derivatives are typically polyfunctional, for example, heptakis-(2,6-di-O-methyl)-β-cyclodextrin and heptakis (2,4,6-tri-O-methyl)-β-cyclodextrin. Other suitable derivatives are hydroxy-β-cyclodextrin and hydroxyethyl-β-cyclodextrin.

### The Complex

The term "complex" is used herein to characterize the actual product obtained by Applicant, and is supported by the literature and Applicant's experimental data. Thus, cyclodextrins, as previously mentioned, are known for their ability to form clath-rates, i.e., inclusion compounds with guest molecules. As will be indicated below in the discussion of preparation of the gastroprotective "complexes" herein disclosed, there appears to be a physical interaction between the cyclodextrin host and the antacid guest that supports the conclusion that a "complex" is obtained, rather than a mixture of these two compounds.

Cyclodextrin compounds are generally represented as having a toroidal molecular structure -- that is, a ring compound having an internal void. Applicant believes, although does not wish to be limited by such belief, that the internal void receives the antacid medicinal agent to form the resulting product that is characterized herein as the complex.

Inasmuch as a particular cyclodextrin provides a specifically defined internal void, the selection of the cyclodextrin to be used in order to maximize the gastroprotective benefit of the complex is dependent upon the antacid agent to be used. Moreover, the properties of the cyclodextrin may be altered by the type and degree of substitution, if any.

The optimization of the resulting gastroprotective complex to embrace maximally effective products may be made by one of ordinary skill in the art by experimentation in accordance with the present disclosure, and especially the examples.

The complexes of the present invention may be best defined in terms of the cyclodextrin-antacid mole ratio. Thus, the degree of gastroprotection for a given dose of the complex appears to be best at a cyclodextrin:antacid mole ratio of about 1:1. Increasing the CD:antacid mole ratio above 1:1 does not appear to provide substantially better gastroprotection, while lowering the mole ratio reduces the level of protection. Suitable CD:antacid mole ratios, then, would generally be in the range of from about 1:2 to about 2:1, with a preferred.ratio of from about 1:1.5 to about 1.5:1, with the most preferred ratio being about 1:1.

The unit dose amount of the complex will be such as to provide an effective gastroprotectant benefit. A unit dose refers to a given weight of the product of the present invention, which may be divided for the convenience of the patient into more than one, usually two, equal amounts, i.e, two tablets. Generally, a unit dose of the complex will contain from about 0.1 to about 25, preferably from about 1 to about 15, most preferably from about 4 to about 10 g/unit dose, the level of the cyclodextrin host molecule being sufficient to provide said complex having the aforementioned mole ratio of the antacid and cyclodextrin constituents.

The unit dose amount may also be stated in terms of the body weight of the patient. Accordingly, the complex contains generally from about 0.001 to about 2.5, preferably from about 0.05 to about 0.75, most preferably from about 0.2 to about 0.67 g/kg body weight of the patient per day. The complex would be dosed at periodic intervals, typically from 1 to 6 times per day, preferably 3 to 6 times per day, to provide such daily dose.

The complex of the present invention may comprise separately formulated complexes using one or more cyclodextrins and/or one or more antacids. The complex of the present invention may also comprise in situ complexation of one or more cyclodextrins with one or more antacids, provided that structured complexes are obtained.

The complex is preferably in the form of a tablet administered orally. The tablet may be a conventional compressed tablet suitable for swallowing whole, but may be chewable or dissolvable in the mouth. The complex may also be administered in powder form, which powder may be taken as a solid or in solution. Also suitable are caplets, powdercontaining capsules, caplet-containing gelatin capsules (e.g., "gelcaps") and other conventional solid dosage forms. It is also possible to administer a solution of the complex parenterally.

### Preparation

The complex may be prepared by forming a solution of cyclodextrin and antacid, followed by stirring until the solution is clear. The solution typically has a concentration of about 10 to about 100, preferably from about 25 to about 75 moles cyclodextrin/liter, and typically has a concentration of from about 10 to about 100, preferably from about 30 to about 80 moles antacid/liter. Alternatively, separate cyclodextrin and antacid solutions may be made and then combined, the combined solution typically having antacid and cyclodextrin concentrations per above.

The solution is stirred at room temperature until clearing. Typically, this will be several hours, but may be several days. The resulting admixture is then shell-frozen at a suitably low temperature, typically between about -50 to about -80°C, preferably from about -60 to about -70°C. The fused mass is then freeze-dried under vacuum at about 1 to about 100, preferably from about 10 to-about 20 millitorrs to obtain the complex.

The complex may then be made into unit doses, for example, a given weight of granular complex or a given weight of a tablet. Applicant has found that an aqueous mixture of the complex thus obtained also provides the intended gastroprotective benefit when orally administered.

### Compositions

The gastroprotective complex may be used as a granular, tablet or solution product obtained upon freeze-drying. It is also within the scope of the present invention to employ one or more excipients in the preparation of a complex-containing medicament. Suitable excipients are:
- Acidifying agents:: acetic acid and citric acid
- Alkalizing agent:: diethanolamine, potassium hydroxide, sodium borate and sodium carbonate
- Antimicrobial agent:: potassium sorbate and sorbic acid
- Antioxidant:: sodium bisulfite
- Binder:: ethyl cellulose, gelatin and guar gum
- Buffering agent:: potassium phosphate and tartaric acid
- Chelating agent:: edetate disodium
- Diluent:: microcrystalline cellulose, mannitol, calcium sulfate and colloidal silicon dioxide
- Disintegrant:: calcium carboxymethyl cellulose, sodium Croscarmello, guar gum, calcium silicate, sodium carboxymethylcellulose, hydroxypropylmethylcellulose, polyethylene glycol, starch, sodium starch and glycolate
- Plasticizer:: glycerin, propylene glycol and citric acid
- Preservative:: benzalkonium chloride and sodium propionate
- Suspending agent:: acacia, agar, Carbopol, hydroxypropyl methylcellulose, pectin, polypropylene glycol, tragacanth and xanthan gum
- Lubricant:: Carbopol, glycol distearate, colloidal silicon dioxide
- Vehicle:: ethyl oleate, mineral oil and polyethylene glycol
- Viscosity increasing agent:: sodium carboxymethyl cellulose, hydroxypropyl methyl cellulose and polyvinyl alcohol

The complexes of the present invention may be employed with one or more additional pharmaceutical actives. In particular, the coadministration of a pharmaceutical active having known gastrointestinal contraindications with the complex of the present invention is an especially desirable embodiment of the present invention. Thus, for example, a composition containing aspirin and the complex of the present invention would protect the gastrointestinal tract from the gastric irritation aspirin is known to cause. In addition to aspirin, other nonsteroidal antiinflammatory drugs are known to cause gastric irritation, for example, ibuprofen, naproxen, ketoprofen, indomethacin, tolmetin and meclafenamate.

The complex may also be coadministered with other pharmaceutical actives that are prone to cause gastric irritation, e.g., potassium supplements.

The compositions of the present invention will contain the known dosage amount of the pharmaceutical active, and an effective dosage amount of the complex. Typically, the amount of the complex used will be the same as previously indicated. In the case of aspirin or other NSAID, a typical formulation will comprise from about 25 to about 2000, preferably from about 100 to about 1000 mg of the NSAID and from about 0.1 to about 20, preferably from about 1 to about 15 g of the complex.

When used alone the complex will protect the GI tract from the effects of alcohol, caffeine, tobacco and the like, which are known gastric irritants. When used following the taking of ethanol, caffeine or tobacco, the complex of the present invention will ameliorate further damage, and accordingly hasten the onset of healing.

The present invention is further illustrated by the examples below, which are not intended to be limiting of the scope of the invention.

### Example 1

A magnesium oxide-cyclodextrin complex was prepared as follows: 25 g α-cyclodextrin (25.7 m moles) were added, with stirring, to 500 g water (reagent grade, 18 megohms resistivity) at room temperature. Following dissolution of the cyclodextrin, 1.21 g MgO (30 m moles) was admixed with stirring, which was continued for about 3 days. The solution was then shell frozen at about -70°C, followed by freeze drying for about two days at a pressure of 20 millitorr. The resultant solid was divided into dosages containing about 15 mEq MgO.

The product obtained above was tested as follows: 10 beagles were fasted overnight. At zero time, the stomach of each animal was endoscopically examined to assure a normal mucosa. Encapsulated compositions containing about 15 mEq MgO in the freshly prepared MgO-CD complex and 975 mg aspirin (ASA) were administered orally and flushed with 50 ml water.

At two and four hours after administration of the encapsulated composition, the stomachs of the animals were endoscopically reexamined for gastric irritation. Irritation was measured according to a scale of 0 to 7, wherein zero was the condition of the mucosa for a normal stomach and a value of 7 represents massive hemorrhagic damage.

In addition, a sample of gastric fluid was obtained from the antrum, and its general appearance noted and its pH measured.

Three control tests were also conducted. In Control A the animals were administered 975 mg ASA. In Control B the animals were administered 6.27 mg α-CD and 975 aspirin and in Control C the animals were administered 15 mEq MgO and 975 aspirin.

The results were as follows:

| Test | n | % Protection | pH |
|---|---|---|---|
| Control A | 20 | 0 | 3.74 ± 0.44 |
| Control B | 4 | 17.4 ± 11.8 | 5.02 ± 0.87 |
| Control C | 4 | 26.6 ± 16.7 | 3.12 ± 0.62 |
| MgO-CD Complex | 10 | 83.5 ± 8.8 | 6.19 ± 0.45 |

wherein n is the number of animals, % Protection is the difference in irritation scores (on the scale of 0 to 7) between Control A and the MgO-CD complex divided by the irritation score for Control A multiplied by 100, and pH is the pH of the antrum fluid sample. The complex of the present invention is seen to have provided a substantial increase in percent protection coupled with a marked increase in pH over the controls.

### Example 2

The procedure of Example 1 was followed, except the mixture of α-cyclodextrin and MgO was stirred for only one hour prior to shell freezing. The resultant solid was formulated with ASA into an encapsulated composition at a 15 mEq MgO level, as in Example 1. Percent protection was 63.3 ± 18.3% (n = 2; pH = 5.15 ± 0.25).

### Example 3

Same as Example 2, except the cyclodextrin and MgO were solubilized in water at 50°C, and thereafter shell frozen. The percent protection was 81.7 ± 0% (n = 2; pH = 6.55 ± 1.15).

### Example 4

Same as Example 3, except that the hot (50°C) CD-MgO solution obtained after mixing for one hour was poured into a glass baking dish and allowed to evaporate at room temperature under a hood. Percent protection was 81.7 ± 0% (n = 2; pH = 5.15 ± 1.75).

### Example 5

To 250 g of a 0.1 N HCl solution were added 0.6 g MgO (15 m moles) with stirring. After three days 12.5 g α-CD (12.85 m moles) was admixed with additional stirring for one day. The solution was shell-frozen and freeze-dried as in Example 1. The resulting product was formulated with 975 mg ASA and sufficient MgO-CD complex to provide a composition containing 2.5 mEq MgO. Percent protection was 81.7 ± O (n = 2; pH = 3.25 ± 0.95).

### Example 6

Same as Example 1, but with four-day stirring. Solution is not shell-frozen nor freeze-dried. Dosed as a solution. Percent protection is 45.0 ± 0% (n = 1; pH = 3.9 ± 0).

### Example 7

Same as Example 1, except the solid product was reconstituted with water and dosed as a solution. Percent protection = 81.7 ± 0% (n = 2; pH = 7.05 ± 0.35).

### Example 8

To 250 g water, 12.5 g (15 m moles) α-CD were added with stirring for 30 minutes, followed by addition of 1.21 g MgO (30 m moles) with stirring, which was continued for four days. The solution was then shell-frozen and freeze-dried, as in Example 1. Percent protection was 93.9 ± 6.1% (n = 3; pH - 7.77 ± 0.09).

### Example 9

Same as Example 1, except shell-frozen at -56°C and freeze-dried. Percent protection was 63.3 ± 0% (n = 1; pH = 6.3).

### Example 10

Same as Example 8, except solution was made with 17.025 g β-CD (15 m moles) and 0.605 g MgO (15 m moles). Percent protection was 90.8 ±9.2% (n = 2; pH = 4.90 ± 1.2).

### Example 11

To 250 g water (reagent grades), 2.9187g α-CD (3 m moles) were added with stirring. Upon dissolution of 1.457 g MgCO₃ (3 m moles) were added, with stirring over three to four days. The solution was shell-frozen and freeze-dried as in Example 1. Testing at an MgCO₃ level of 15 mEq was conducted as in Example 1, the percent protection being 72.5 ± 27.5% (n = 2; pH = 4.35 ± 2.75). Use of MgCO₃ alone as a control provided a percent protection of 2.1 ± 6.1% (n = 3; pH = 3.33 ± 0.81).

### Example 12

Same as Example 11, except 5.837 g α-CD (6 m moles) and 1.457 g MgCO₃ (3 m moles) were incorporated into solution. Percent protection was 45.0 ±0% (n = 2; pH = 6.35 ± 0.15).

### Example 13

To 250 g water (reagent grade), 3.4057 g β-cyclodextrin (3 m moles) were added. Upon dissolution 1.4571 g MgCO₃ (3 m moles) were added, with stirring for three to four days. the solution was shell-frozen and freeze-dried, with testing of product at 15 mEq MgCO₃, as in Example 11. Percent protection was 45.0 ± 0% (n = 2; pH = 3.75 ± 0.15).

### Example 14

To 250 g water, 14.5935 g α-cyclodextrin (15 m moles) was added, followed by addition of 1.5015 g CaCO₃ (15 m moles), with stirring for three to four days. Remainder of processing and testing followed Example 1. Percent protection was 81.7 ± 0% (n = 2; pH = 6.3 ± 0.1). Use of CaCO₃ alone as a control provided a percent protection of 3.0 ± 5% (n = 3; pH = 4.17 ± 0.90).

### Example 15

Same as Example 14, except 17.0255 g β-CD (15 m moles) and 3.0035 g CaCO₃ (30 m moles) were incorporated into solution. Percent protection was 81.7 ± 0% (n = 1; pH = 7.1 ± 0).

### Example 16

To 250 g water, 9.729 g α-cyclodextrin (10 m moles) were added, followed by addition of 780 mg Al(OH)₃ (10 m moles) with stirring for three to four days. With the further processing and testing of Example 1, percent protection was 40.4 ± 21.7% (n = 4; pH = 3.75 ± 0.98). Use of Al(OH)₃ alone as a control provided a percent protection of 10.8 ± 4.6% (n = 4; pH = 4.72 ± 0.85).

### Example 17

To 500 g water (reagent grade), 26.5155 g α-cyclodextrin (27.2341 m moles) were added, followed by the addition, with stirring, of 533.7 mg MgO (13.266 m moles), 333.6 mg MgCO₃ (0.6868 m moles) and 1.3344 g CaCO₃ (13.33067 m moles). Stirring for three to four days followed. Remaining processing and testing was the same as Example 1. Percent protection was 72.5 ± 15.9% (n = 4; pH = 6.35 ± 0.46).

### Example 18

To 250 g water, 26.5155 g α-CD (27.2541 m moles) was added followed by addition of a 2.647 g (60 mEq) alkaline mix containing 533.7 mg MgO (13.266 m moles), 333.6 mg MgCO₃ (0.6868 m moles) and 1.3344 g CaCO₃ (13.33067 m moles) and 0.4453 g starch. Processing and testing as in Example 1 followed. Percent Protection was 90.8 ± 9.2% (n = 2, pH = 6.05 ± 0.55). The alkaline mix alone as a control provided a percent protection of 16.6 ± 11.4% (n = 11; pH = 2.90 ± 0.33).

## Claims

1. A gastroprotective product comprising an antacid agent guest molecule having a cation selected from aluminum, magnesium and calcium, and a cyclodextrin host molecule to which said antacid agent guest molecule is bound, said product being suitable for mammalian administration in an amount effective to provide gastrointestinal cytoprotection.

2. The gastroprotective product of Claim 1 wherein the cyclodextrin to antacid mole ratio is from about 1:2 to about 2:1.

3. The gastroprotective product of Claim 1 or Claim 2 wherein the antacid agent host molecule contains an anion selected from carbonate, trisilicate and hydroxide.

4. The gastroprotective product of Claim 1 or Claim 2 wherein the antacid agent host molecule is selected from magnesium oxide and magnesium-aluminum hydroxide gels.

5. A gastroprotective product suitable for oral administration to a mammal comprising a cyclodextrin-antacid agent inclusion compound containing from about 0.5 to about 2 moles antacid agent per mole of cyclodextrin, a unit dose of said product containing from about 0.225 to about 60 mEq of said antacid, whereby administration of said unit dose is effective to provide gastrointestinal cytoprotection

6. The gastroprotective product of Claim 5 wherein the unit dose contains from about 2.25 to about 30 Meq antacid.

7. The gastroprotective product of Claim 5 or claim 6 wherein the cyclodextrin-antacid agent mole ratio is from about 1:1.5 to about 1.5:1, said unit dose containing from about 2.25 to about 30 mEq of the antacid.

8. The gastroprotective product of any of Claims 5 to 7 wherein the cyclodextrin-antacid agent mole ratio is about 1:1, said unit dose containing from about 10 to about 22.5 mEq of the antacid.

9. The gastroprotective product of any of Claims 5 to 8 wherein the antacid agent is selected from aluminum hydroxide, aluminum carbonate, magnesium hydroxide, magnesium oxide, magnesium carbonate, magnesium trisilicate, calcium carbonate, magnesium-aluminum hydroxide gels, and mixtures thereof.

10. the gastroprotective product of any of Claims 5 to 8 wherein the antacid agent is magaldrate.

11. The gastroprotective product of any of Claims 5 to 8 wherein the cyclodextrin is α-cyclodextrin and the antacid is magnesium hydroxide.

12. The gastroprotective product of any of Claims 5 to 8 wherein the cyclodextrin is β-cyclodextrin and the antacid is aluminum carbonate.

13. the gastroprotective product of any preceding claim wherein the cyclodextrin is selected from α-, β- and γ-cyclodextrins, and their C₁ to C₄ alkyl and hydroxy-alkyl derivative.

14. A medicinal composition suitable for oral administration to a mammal comprising in combination a pharmaceutically active agent in effective amount, gastrointestinal irritation being occasioned by the independent administration of said pharmaceutically active agent, and the gastroprotective product of any preceding claim, whereby the gastrointestinal irritation occasioned by said pharmaceutically active agent is diminished.

15. The composition of Claim 14 wherein said pharmaceutically active agent is an analgesic.

16. The composition of Claim 15 wherein the analgesic is selected from aspirin, ibuprofen, naproxen, ketoprofen, indomethacin, tolmetin and meclofenamate.

17. A process for making a gastroprotective product having an antacid guest molecule and a cyclodextrin host molecule comprising the steps of freezing an aqueous mixture of the cyclodextrin and the antacid to obtain a fused mass, and then freeze-drying the fused mass to obtain the gastroprotectant product.

18. The process of Claim 17 wherein said aqueous mixture has an antacid concentration of from about 10 to about 100 moles per liter and a cyclodextrin concentration of from about 10 to about 100 moles liter.

19. The process of Claim 18 further comprising the steps of solubilizing the antacid in aqueous media; solubilizing the cyclodextrin in aqueous media, and admixing said solubilized antacid and cyclodextrin solutions to obtain said aqueous cyclodextrin-antacid mixture.

20. The process of Claim 17 wherein said aqueous mixture is rapidly frozen at below about -50°C.

21. The process of any of Claims 17 to 20 wherein the antacid guest molecule and the cyclodextrin host molecule form an inclusion compound.

22. The use in the manufacture of a pharmaceutical for oral administration for alleviating gastrointestinal irritation of a gastroprotective product of any of Claim 1 or 13.

23. The use of Claim 22 wherein said pharmaceutical is in the form of a unit dose suitable for administration from one to six times per day.

24. The use of Claim 23 wherein said pharmaceutical is in the form suitable for administration to provide a daily dose of the gastroprotective product of from 0.001 to about 2.5 g/kg of subject body weight per day.

25. The use of Claim 22 wherein said pharmaceutical is in a form suitable for permitting the gastroprotective product to be coadministered with an agent causing gastroirritation.

26. The use of a cyclodextrin in the manufacture of a pharmaceutical for providing gastrointestinal cytoprotection.
